# EUROPEAN PATENT APPLICATION

(11) **EP 4 548 910 A2**
(43) Date of publication of application: **07.05.2025**
(21) Application number: 24221336.1
(22) Date of filing: 19.12.2024
(51) Int. Cl.: A61K 8/42, A61K 8/49, A61K 31/16, A61K 31/341, A61P 27/04, A61P 27/16, A61Q 11/00, A61Q 19/10

(54) **COMPOUNDS FOR USE ON MUCOSAL TISSUE**

(71) Applicant: CLARIANT INTERNATIONAL LTD, 4132 Muttenz (CH)
(72) Inventor: Back, Ute, 65926 Frankfurt am Main (DE); Riederle, Petra, 87637 Seeg (DE); Grohmann, Jörg, 65926 Frankfurt am Main (DE); Fricke, Tom, 65926 Frankfurt am Main (DE)
(74) Representative: Clariant Produkte (Deutschland) GmbH

(57) **Abstract**

The present invention relates to the use of compound X selected from compounds of Formula (I) 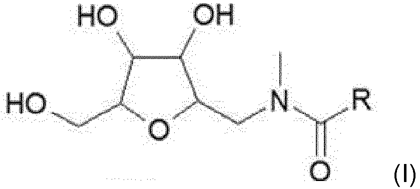 wherein R is selected from saturated hydrocarbon chains having 5 to 23 carbon atoms, unsaturated hydrocarbon chains having 5 to 23 carbon atoms, and mixtures thereof,
compounds of Formula (II) 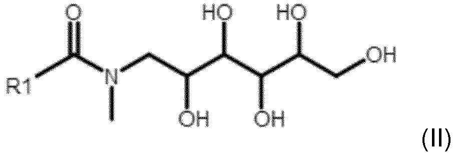 wherein R1 is selected from saturated hydrocarbon chains having 5 to 23 carbon atoms, unsaturated hydrocarbon chains having 5 to 23 carbon atoms, and mixtures thereof,
glyceryl ethers, sorbitan esters, and isosorbide esters
on mucosal tissue.

## Description

The present invention relates to the use of certain compounds on mucosal tissue, to care formulations comprising these compounds, and to the use of the care formulations on mucosal tissue. The present invention also relates to the compounds for use on mucosal tissue and to the care formulations for use on mucosal tissue.

Oral care, intimate care, eye care, ear care, and nasal care are important for general hygiene and overall well-being. Oral care includes, among others, brushing the teeth with toothpaste and rinsing the mouth with mouthwash.

There is an ongoing need for ingredients that can be used in care formulations such as oral care formulations, intimate care formulations, eye care formulations, ear care formulations, nasal care formulations, and lubricants for medical devices or medical tools.

It has been found that certain compounds are compatible with mucosal tissue and can therefore be used in care formulations such as oral care formulations, intimate care formulations, eye care formulations, ear care formulations, nasal care formulations, and lubricants for medical devices or medical tools.

Accordingly, the present invention relates to the use of compound X selected from compounds of Formula (I) 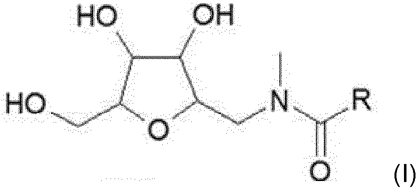 wherein R is selected from saturated hydrocarbon chains having 5 to 23 carbon atoms, unsaturated hydrocarbon chains having 5 to 23 carbon atoms, and mixtures thereof, compounds of Formula (II) 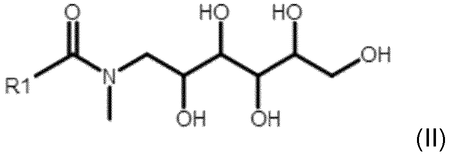 wherein R1 is selected from saturated hydrocarbon chains having 5 to 23 carbon atoms, unsaturated hydrocarbon chains having 5 to 23 carbon atoms, and mixtures thereof,
glyceryl ethers, sorbitan esters, and isosorbide esters
on mucosal tissue.

Advantageously, the compounds used in the present invention have a high renewable carbon content, i.e. they are sustainable. The compounds used in the present invention can be incorporated into care formulations such as oral care formulations, intimate care formulations, eye care formulations, ear care formulations, nasal care formulations, and lubricants for medical devices or medical tools. Advantageously, the compounds can be used to protect or prevent the mucosal tissue from damage. Advantageously, the compounds can be used to protect, maintain or increase the tissue viability and/or tissue integrity of the mucosal tissue.

According to the invention, compound X is used on mucosal tissue.

Preferably, the mucosal tissue is selected from oral mucosal tissue, intimate mucosal tissue, eye mucosal tissue, ear mucosal tissue (including eardrum), and nasal mucosal tissue. In a preferred embodiment, the mucosal tissue is selected from oral mucosal tissue and intimate mucosal tissue. In a particularly preferred embodiment, the mucosal tissue is oral mucosal tissue. In a preferred embodiment, the mucosal tissue is intimate mucosal tissue. In a preferred embodiment, the mucosal tissue is selected from intimate mucosal tissue, eye mucosal tissue, ear mucosal tissue (including eardrum), and nasal mucosal tissue.

Particularly preferably, compound X is applied topically to the mucosal tissue.

According to the invention, compound X is selected from
compounds of Formula (I) 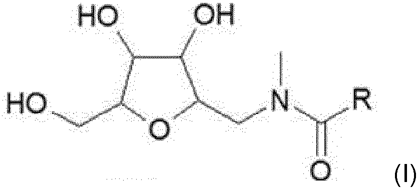
wherein R is selected from saturated hydrocarbon chains having 5 to 23 carbon atoms, unsaturated hydrocarbon chains having 5 to 23 carbon atoms, and mixtures thereof,
compounds of Formula (II) 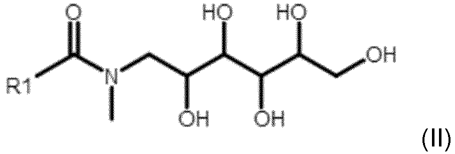
wherein R1 is selected from saturated hydrocarbon chains having 5 to 23 carbon atoms, unsaturated hydrocarbon chains having 5 to 23 carbon atoms, and mixtures thereof,
glyceryl ethers, sorbitan esters, and isosorbide esters.

Preferably, compound X is selected from compounds of Formula (I) and compounds of Formula (II).

In a preferred embodiment, compound X is selected from compounds of Formula (I).

Preferably, R in Formula (I) is selected from saturated hydrocarbon chains having 5 to 17 carbon atoms, unsaturated hydrocarbon chains having 5 to 17 carbon atoms, and mixtures thereof. More preferably, R in Formula (I) is selected from saturated hydrocarbon chains having 5 to 13 carbon atoms, unsaturated hydrocarbon chains having 5 to 13 carbon atoms, and mixtures thereof. Particularly preferably, R in Formula (I) is selected from saturated hydrocarbon chains having 7 to 9 carbon atoms, unsaturated hydrocarbon chains having 7 to 9 carbon atoms, and mixtures thereof.

In at least one embodiment, R in Formula (I) is selected from -(CH₂)₆CH₃, - (CH₂)₈CH₃, and mixtures thereof; or R in Formula (I) is selected from -(CH₂)₁₀CH₃, -(CH₂)₁₂CH₃, and mixtures thereof; or R in Formula (I) is selected from - (CH₂)₇CH=CH₂, -(CH₂)₇CH=CHCH₂CH₃, and mixtures thereof; or the R-C=O residue in Formula (I) is derived from coconut fatty acids; or the R-C=O residue in Formula (I) is derived from soybean fatty acids.

In at least one embodiment, R in Formula (I) is selected from -(CH₂)₆CH₃, - (CH₂)₈CH₃, and mixtures thereof. In at least one embodiment, R in Formula (I) is selected from -(CH₂)₁₀CH₃, -(CH₂)₁₂CH₃, and mixtures thereof. In at least one embodiment, R in Formula (I) is selected from -(CH₂)₇CH=CH₂, - (CH₂)₇CH=CHCH₂CH₃, and mixtures thereof. In at least one embodiment, the R-C=O residue in Formula (I) is derived from coconut fatty acids. In at least one embodiment, the R-C=O residue in Formula (I) is derived from soybean fatty acids.

As used herein, the expression "the R-C=O residue in Formula (I) is derived from coconut fatty acids" preferably means that the carbon chain length distribution in the R-C=O residue in Formula (I) corresponds to the carbon chain length distribution of the fatty acids (e.g. bound as triglycerides) in coconut oil.

As used herein, the expression "the R-C=O residue in Formula (I) is derived from soybean fatty acids" preferably means that the carbon chain length distribution in the R-C=O residue in Formula (I) corresponds to the carbon chain length distribution of the fatty acids (e.g. bound as triglycerides) in soybean oil.

Preferably, the weight ratio of compounds according to Formula (I) wherein R is - (CH₂)₆CH₃ and compounds according to Formula (I) wherein R is -(CH₂)₈CH₃ is from 1:9 to 9:1, preferably from 3:7 to 7:3. Preferably, the weight ratio of compounds according to Formula (I) wherein R is -(CH₂)₁₀CH₃ and compounds according to Formula (I) wherein R is -(CH₂)₁₂CH₃ is from 1:9 to 9:1, preferably from 3:7 to 7:3. Preferably, the weight ratio of compounds according to Formula (I) wherein R is -(CH₂)₇CH=CH₂ and compounds according to Formula (I) wherein R is -(CH₂)₇CH=CHCH₂CH₃ is from 1:9 to 9:1, preferably from 3:7 to 7:3.

Advantageously, the compound of Formula (I) is soluble in water. Therefore, the compound of Formula (I) is suitable for use in mouth wash formulations.

Compounds of Formula (I) are described in, e.g., WO 2018/002100 and EP application number EP21200587.0.

In a preferred embodiment, compound X is selected from compounds of Formula (II).

Preferably, R1 in Formula (II) is selected from saturated hydrocarbon chains having 5 to 17 carbon atoms, unsaturated hydrocarbon chains having 5 to 17 carbon atoms, and mixtures thereof. More preferably, R1 in Formula (II) is selected from saturated hydrocarbon chains having 7 to 13 carbon atoms, unsaturated hydrocarbon chains having 7 to 13 carbon atoms, and mixtures thereof. Particularly preferably, R1 in Formula (II) is selected from saturated hydrocarbon chains having 11 to 13 carbon atoms, unsaturated hydrocarbon chains having 11 to 13 carbon atoms, and mixtures thereof.

Also preferably, the R1-C=O residue in Formula (II) is derived from caprylic acid, capric acid, lauric acid, myristic acid, palmitic acid, stearic acid, oleic acid, linoleic acid, linolenic acid, coconut fatty acids, or mixtures thereof. Also preferably, the R1-C=O residue in Formula (II) is derived from 9-decenoic acid, 9-dodecenoic acid, or mixtures thereof.

Particularly preferred compounds of Formula (II) are capryloyl/caproyl methyl glucamide, lauroyl/myristoyl methyl glucamide, cocoyl methyl glucamide, sunfloweroleyl methyl glucamide, or mixtures thereof. Such compounds are commercially available from Clariant (GlucoTain^{®} Clear, GlucoTain^{®} Plus, GlucoTain^{®} Flex, GlucoTain^{®} Care, GlucoTain^{®} Sense).

In one embodiment, compound X is selected from glyceryl ethers.

Preferably, the glyceryl ethers are mono- or diethers of glycerin and one or more C6-C20 fatty alcohols. More preferably, the glyceryl ethers are mono- or diethers of glycerin and one or more C8-C14 fatty alcohols. Particularly preferably, the glyceryl ethers are monoethers of glycerin and one or more C8 fatty alcohols.

In at least one embodiment, the glyceryl ethers are selected from ethylhexylglycerin, methylheptylglycerin, caprylyl glyceryl ether, and mixtures thereof.

In one embodiment, compound X is selected from sorbitan esters and isosorbide esters.

In one embodiment, compound X is selected from sorbitan esters.

Preferably, the sorbitan esters are mono-, di- or triesters of sorbitan and one or more C6-C20 fatty acids. More preferably, the sorbitan esters are mono- or diesters of sorbitan and one or more C8-C14 fatty acids. Particularly preferably, the sorbitan esters are mono- or diesters of sorbitan and caprylic acid.

Preferably, the sorbitan esters are selected from sorbitan caprylate, sorbitan stearate, sorbitan olivate, sorbitan oleate, sorbitan caprate, sorbitan laurate, sorbitan myristate, sorbitan caproate, and mixtures thereof. Particularly preferably, the sorbitan ester is sorbitan caprylate.

In one embodiment, compound X is selected from isosorbide esters.

Preferably, the isosorbide esters are mono- or diesters of isosorbide and one or more C6-C20 fatty acids. More preferably, the isosorbide esters are mono- or diesters of isosorbide and one or more C8-C14 fatty acids. Particularly preferably, the isosorbide esters are mono- or diesters of isosorbide and caprylic acid.

Preferably, the isosorbide esters are selected from isosorbide caprylate, isosorbide stearate, isosorbide olivate, isosorbide oleate, isosorbide caprate, isosorbide laurate, isosorbide myristate, isosorbide caproate, and mixtures thereof. Particularly preferably, the isosorbide ester is isosorbide caprylate.

Advantageously, compound X is suitable for use in toothpaste formulations.

The present invention also relates to compound X as defined herein for use on mucosal tissue. Preferred embodiments of compound X are described further above. Preferably, the mucosal tissue is selected from oral mucosal tissue, intimate mucosal tissue, eye mucosal tissue, ear mucosal tissue (including eardrum), and nasal mucosal tissue. Particularly preferably, compound X is applied topically to the mucosal tissue.

In a preferred embodiment, compound X is used to protect or prevent the mucosal tissue from damage. In a preferred embodiment, compound X is used to protect, maintain or increase the tissue viability and/or tissue integrity of the mucosal tissue. In at least one embodiment, compound X is used to protect, maintain or increase the tissue viability of the mucosal tissue. In at least one embodiment, compound X is used to protect, maintain or increase the tissue integrity of the mucosal tissue.

The present invention also relates to a care formulation comprising from 0.1 to 20 wt.-%, preferably from 0.2 to 10 wt.-%, more preferably from 0.3 to 5 wt.-%, even more preferably from 0.4 to 3 wt.-%, particularly preferably from 0.5 to 2 wt.-%, for example from 0.8 to 1.5 wt.-%, based on the total weight of the care formulation, of compound X as defined herein,
wherein the care formulation is selected from an oral care formulation, intimate care formulation, eye care formulation, ear care formulation, nasal care formulation, and lubricant for medical devices or medical tools.

Preferred embodiments of compound X are described further above.

According to the invention, the care formulation is selected from an oral care formulation, intimate care formulation, eye care formulation, ear care formulation, nasal care formulation, and lubricant for medical devices or medical tools. In a preferred embodiment, the care formulation is selected from an oral care formulation, intimate care formulation, eye care formulation, ear care formulation, and nasal care formulation. In a more preferred embodiment, the care formulation is selected from an oral care formulation and intimate care formulation. In a particularly preferred embodiment, the care formulation is an oral care formulation. In a preferred embodiment, the care formulation is an intimate care formulation. In a preferred embodiment, the care formulation is a lubricant for medical devices or medical tools. In a preferred embodiment, the care formulation is selected from an intimate care formulation, eye care formulation, ear care formulation, nasal care formulation, and lubricant for medical devices or medical tools.

Preferably, the care formulation is for human care or animal care. Particularly preferably, the care formulation is for human care. Also particularly preferably, the care formulation is for animal care.

Preferably, the oral care formulation is selected from a toothpaste, oral gel, mouth wash, tooth powder, oral spray, oral care wipes, dental wipes, dental floss, and chewing gum. In a preferred embodiment, the oral care formulation is selected from a toothpaste and mouth wash. In a particularly preferred embodiment, the oral care formulation is a toothpaste. In another particularly preferred embodiment, the oral care formulation is a mouth wash. Preferably, the intimate care formulation is selected from an intimate cleansing formulation, intimate deodorant, intimate lubricant, and intimate wipes. In one embodiment, the intimate care formulation is selected from an intimate cleansing formulation, intimate deodorant, and intimate wipes. Preferably, the eye care formulation is selected from an eye cleansing formulation, lense cleaning formulation, and eye makeup remover. Preferably, the ear care formulation is an ear cleansing formulation. Preferably, the nasal care formulation is a moisturizing nasal care formulation, which is preferably in the form of a spray, gel, cream, or ointment. Preferably, the lubricant for medical devices or medical tools is an ultrasound gel.

Optionally, the care formulation comprises a (further) antimicrobial agent.

In at least one embodiment, the antimicrobial agent is selected from aromatic alcohols, organic acids and salts thereof, hydroxamic acids and salts thereof, hydroxyacetophenone, compounds according to Formula (P), alkyl diols, halogenated compounds, isothiazolinones, and mixtures thereof, wherein Formula (P) is as follows: 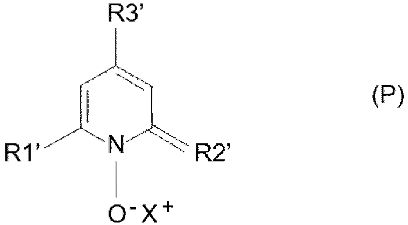 wherein
- R1': is H, an unsubstituted or halogen-substituted, branched or unbranched C₁-C₂₀-alkyl radical, an unsubstituted or halogen-substituted C₅-C₈-cycloalkyl radical, an unsubstituted or halogen-substituted C₆-C₁₀-aryl radical or an unsubstituted or halogen-substituted, branched or unbranched C₇-C₂₀-aralkyl radical;
- R2': is O or S;
- R3': is H or a C₁-C₄-alkyl radical;
- X⁺: is a cation.

Preferably, R3' is methyl.

In at least one embodiment, the aromatic alcohols are selected from benzyl alcohol, phenoxyethanol, veratryl alcohol, propylene phenoxyethanol, phenethyl alcohol, phenylpropanol, vanillin, 2-methyl-1-phenyl-2-propanol, and mixtures thereof.

In at least one embodiment, the organic acids and salts thereof are selected from benzoic acid, sorbic acid, dehydroacetic acid, lactic acid, salicylic acid, p-anisic acid, undecylenic acid, glycolic acid, propionic acid, levulinic acid, and mixtures thereof.

In at least one embodiment, the hydroxamic acid is selected from hydroxamic acids of Formula (III) 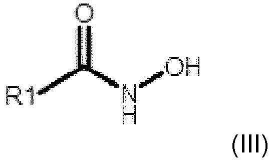 wherein R¹ is selected from saturated hydrocarbon chains having 5 to 23 carbon atoms, unsaturated hydrocarbon chains having 5 to 23 carbon atoms, and mixtures thereof.

Preferably, R¹ in Formula (III) is selected from saturated hydrocarbon chains having 5 to 17 carbon atoms, unsaturated hydrocarbon chains having 5 to 17 carbon atoms, and mixtures thereof. More preferably, R¹ in Formula (III) is selected from saturated hydrocarbon chains having 5 to 13 carbon atoms, unsaturated hydrocarbon chains having 5 to 13 carbon atoms, and mixtures thereof. Particularly preferably, R¹ in Formula (III) is selected from saturated hydrocarbon chains having 7 carbon atoms, unsaturated hydrocarbon chains having 7 carbon atoms, and mixtures thereof.

Preferred hydroxamic acids are selected from caprylhydroxamic acid, hexanohydroxamic acid, caprohydroxamic acid, laurohydroxamic acid, and mixtures thereof. A particularly preferred hydroxamic acid is caprylhydroxamic acid. Caprylhydroxamic acid may also be referred to as caprylohydroxamic acid or octanohydroxamic acid. Hydroxamic acids are described in, e.g., EP2224973.

Examples of salts of hydroxamic acids are alkali metal salts of hydroxamic acids (e.g. sodium salts of hydroxamic acids or potassium salts of hydroxamic acids) or alkaline earth metal salts of hydroxamic acids (e.g. magnesium salts of hydroxamic acids or calcium salts of hydroxamic acids).

In at least one embodiment, the compound according to Formula (P) is selected from 2-hydroxypyridine-N-oxide, 2-pyridinethiol-1-oxide and salts thereof, 1-hydroxy-4-methyl-6-(2,4,4-trimethylpentyl)-2(1H)-pyridone and salts thereof (preferably the monoethanolamine salt), and mixtures thereof. Formula (P) discloses and encompasses the tautomeric equivalents of these compounds, since an equilibrium always exists. In at least one embodiment, the compound according to Formula (P) is piroctone olamine (Octopirox).

In at least one embodiment, the alkyl diols are selected from 1,2-pentanediol, 1,2-hexanediol, 1,6-hexanediol, 1,2-octanediol, 1,2-heptanediol, 1,2-decanediol, methylpropanediol, and mixtures thereof.

In at least one embodiment, the halogenated compounds are selected from chlorhexidine and salts thereof, triclosan, chlorphenesin, trichlorcarban, chloroxylenol, bronopol, climbazole, and mixtures thereof.

In at least one embodiment, the isothiazolinones are selected from methylisothiazolinone, methylchloroisothiazolinone, benzylisothiazolinone, and mixtures thereof.

In at least one embodiment, the antimicrobial agent is selected from aromatic alcohols, organic acids and salts thereof, hydroxamic acids and salts thereof, hydroxyacetophenone, hydroxypyridones, alkyl diols, halogenated compounds, isothiazolinones, and mixtures thereof.

In at least one embodiment, the antimicrobial agent is selected from phenoxyethanol, benzyl alcohol, phenethyl alcohol, benzoic acid and salts thereof, caprylhydroxamic acid, hydroxyacetophenone, piroctone olamine, and mixtures thereof.

In a preferred embodiment, the antimicrobial agent is 1,2-octanediol, in particular bio-based 1,2-octanediol, for example bio-1,2-octanediol as disclosed in WO 2019/152569.

In one embodiment, the care formulation, preferably oral care formulation comprises a solvent. The solvent is preferably selected from water, propylene glycol, isopropanol, and mixtures thereof.

In one embodiment, the care formulation, preferably oral care formulation comprises a humectant. The humectant is preferably selected from sorbitol, glycerol, xylitol, and mixtures thereof. The humectant may, for example, be present in an amount of from 10 to 70 wt.-%, preferably from 30 to 60 wt.-%, for example from 40 to 55 wt.-%, based on the total weight of the care formulation, preferably oral care formulation.

In one embodiment, the care formulation, preferably oral care formulation comprises an abrasive. The abrasive is preferably selected from silica abrasives, hydrated silica, hydrated alumina, calcium abrasives, tricalcium phosphate, hydroxyapatite, dicalcium phosphate dihydrate, calcium pyrophosphate, calcium carbonate, sodium metaphosphate, potassium metaphosphate, aluminum silicate, calcined alumina, bentonite, sodium bicarbonate, and mixtures thereof. The abrasive may, for example, be present in an amount of from 10 to 30 wt.-%, preferably from 15 to 25 wt.-%, based on the total weight of the care formulation, preferably oral care formulation.

In one embodiment, the care formulation, preferably oral care formulation comprises an anticalculus agent. The anticalculus agent is preferably selected from phosphates, polyphosphates, pyrophosphates, e.g. tetrasodium pyrophosphate, polyaminopropanesulfonic acid, hexametaphosphate salts, zinc citrate trihydrate, polypeptides, polyolefin sulfonates, polyolefin phosphates, diphosphonates, and mixtures thereof. The anticalculus agent may, for example, be present in an amount of from 0.01 to 20 wt.-%, preferably from 0.1 to 10 wt.-%, based on the total weight of the care formulation, preferably oral care formulation.

In one embodiment, the care formulation, preferably oral care formulation comprises a fluoride source. The fluoride source is preferably selected from stannous fluoride, sodium fluoride, potassium fluoride, sodium monofluorophosphate, sodium fluorosilicate, ammonium fluorosilicate, amine fluoride, ammonium fluoride, calcium fluoride, aluminum fluoride, fluorohydroxyapatite, and mixtures thereof. The fluoride source is preferably selected from stannous fluoride, sodium fluoride, potassium fluoride, sodium monofluorophosphate, sodium fluorosilicate, ammonium fluorosilicate, amine fluoride, ammonium fluoride, and mixtures thereof. The fluoride source may, for example, be present in an amount of from 0.01 to 10 wt.-%, preferably from 0.1 to 2 wt.-%, based on the total weight of the care formulation, preferably oral care formulation. The fluoride source may, for example, be present in an amount of from 0.01 to 1 wt.-%, preferably from 0.02 to 0.5 wt.-%, more preferably from 0.03 to 0.3 wt.-%, even more preferably from 0.04 to 0.2 wt.-%, particularly preferably from 0.05 to 0.15 wt.-%, based on the total weight of the care formulation, preferably oral care formulation. The fluoride source may, for example, be present in an amount of from 0.01 to 0.15 wt.-%, preferably from 0.02 to 0.15 wt.-%, more preferably from 0.03 to 0.15 wt.-%, even more preferably from 0.04 to 0.15 wt.-%, particularly preferably from 0.05 to 0.15 wt.-%, based on the total weight of the care formulation, preferably oral care formulation. In a preferred embodiment, the care formulation, preferably oral care formulation is a toothpaste, and the toothpaste comprises a fluoride source.

In one embodiment, the care formulation, preferably oral care formulation comprises a polymer. The polymer is preferably selected from polyethylene glycols, polysaccharides, for example cellulose derivatives, for example carboxymethyl cellulose, hydroxymethyl cellulose, ethyl cellulose, microcrystalline cellulose, or polysaccharide gums, for example xanthan gum, guar gum or carrageenan gum, or polyacrylates or polymers based on N-vinylpyrrolidone, and mixtures thereof. The polymer may, for example, be present in an amount of from 0.1 to 10 wt.-%, preferably from 0.5 to 5 wt.-%, based on the total weight of the care formulation, preferably oral care formulation.

In one embodiment, the care formulation, preferably oral care formulation comprises an anionic polymer. The anionic polymer is preferably selected from polycarboxylates, polyacrylic acids, polyacrylates, polyphosphonic acids, crosslinked carboxyvinyl copolymers, and mixtures thereof. The anionic polymer may, for example, be present in an amount of from 1 to 20 wt.-%, preferably from 5 to 15 wt.-%, based on the total weight of the care formulation, preferably oral care formulation.

In one embodiment, the care formulation, preferably oral care formulation comprises a thickening agent. The thickening agent is preferably selected from silica thickening agents, carboxy vinyl polymers, carrageenan, hydroxyethyl cellulose, salts of cellulose ethers such as sodium carboxymethyl cellulose or sodium carboxymethyl hydroxyethyl cellulose, karaya, gum arabic, gum tragacanth, colloidal magnesium aluminum silicate, and mixtures thereof. The thickening agent may, for example, be present in an amount of from 0.1 to 10 wt.-%, preferably from 0.5 to 5 wt.-%, based on the total weight of the care formulation, preferably oral care formulation.

In one embodiment, the care formulation, preferably oral care formulation comprises a foaming agent. The foaming agent is preferably selected from polyethylene glycol, alginate polymers, and mixtures thereof. The foaming agent may, for example, be present in an amount of from 0.1 to 20 wt.-%, preferably from 1 to 10 wt.-%, based on the total weight of the care formulation, preferably oral care formulation.

In one embodiment, the care formulation, preferably oral care formulation comprises a surfactant. The surfactant may, for example, be present in an amount of from 0.1 to 20 wt.-%, preferably from 1 to 10 wt.-%, based on the total weight of the care formulation, preferably oral care formulation. In one embodiment, the surfactant is an anionic surfactant. The anionic surfactant is preferably selected from alkyl sulfates, for example sodium lauryl sulfate, alkyl ether sulfates, for example sodium laureth-2 sulfate, alkyl aryl sulfonates, for example sodium lauryl benzene sulfonate, alkyl sulfoacetates, for example sodium lauryl sulfoacetate, and mixtures thereof. In one embodiment, the surfactant is a sulfur-free anionic surfactant. The sulfur-free anionic surfactant is preferably selected from taurates, for example sodium methyl cocoyl taurate, glycinates, for example sodium cocoyl glycinate, sarcosinates, for example sodium lauroyl sarcosinate, quaternary ammonium compounds, for example benzalkonium chloride or cetylpyridinium chloride, and mixtures thereof. In one embodiment, the surfactant is a non-ionic surfactant, for example an alkyl polyglycoside (APG), preferably an alkyl polyglucoside. In one embodiment, the surfactant is a zwitterionic surfactant, for example a betaine surfactant, for example cocamidopropyl betaine. In one embodiment, the surfactant is a cationic surfactant.

In one embodiment, the care formulation, preferably oral care formulation comprises a buffer or pH adjusting agent. In a preferred embodiment, the care formulation, preferably oral care formulation has a pH in the range of from 4 to 8, preferably from 4.5 to 7.5, more preferably from 5 to 7, particularly preferably from 5 to 6, also particularly preferably from 5.5 to 6.5, also particularly preferably from 6 to 7. In a preferred embodiment, the care formulation, preferably oral care formulation is a toothpaste, and the toothpaste has a pH in the range of from 4 to 8, preferably from 4.5 to 7.5, more preferably from 5 to 7, particularly preferably from 5 to 6, also particularly preferably from 5.5 to 6.5, also particularly preferably from 6 to 7.

In one embodiment, the care formulation, preferably oral care formulation comprises a flavoring agent, sweetening agent, and/or pigment or dye. Examples of flavors are mint, peppermint, spearmint, wintergreen, menthol, or strawberry. An example of a sweetening agent is sodium saccharin. Examples of pigments are titanium dioxide or zinc oxide.

The care formulation, preferably oral care formulation may, for example, be prepared by mixing its ingredients.

The present invention also relates to the use of a care formulation of the present invention on mucosal tissue. Preferred embodiments of the care formulation are described further above. Preferably, the mucosal tissue is selected from oral mucosal tissue, intimate mucosal tissue, eye mucosal tissue, ear mucosal tissue (including eardrum), and nasal mucosal tissue. Particularly preferably, the care formulation is applied topically to the mucosal tissue.

The present invention also relates to a care formulation of the present invention for use on mucosal tissue. Preferred embodiments of the care formulation are described further above. Preferably, the mucosal tissue is selected from oral mucosal tissue, intimate mucosal tissue, eye mucosal tissue, ear mucosal tissue (including eardrum), and nasal mucosal tissue. Particularly preferably, the care formulation is applied topically to the mucosal tissue.

In a preferred embodiment, compound X is used to protect or prevent the mucosal tissue from damage. In a preferred embodiment, compound X is used to protect, maintain or increase the tissue viability and/or tissue integrity of the mucosal tissue. In at least one embodiment, compound X is used to protect, maintain or increase the tissue viability of the mucosal tissue. In at least one embodiment, compound X is used to protect, maintain or increase the tissue integrity of the mucosal tissue.

In this document, the following definitions apply unless specifically stated otherwise. All percentages are by weight (w/w) of the total composition. All ratios are weight ratios. "wt.-%" means percentage by weight.

"Molecular weight" or "M.Wt." or "MW" and grammatical equivalents mean the number average molecular weight.

"Viscosity" is measured at 25°C using a HAAKE Rotation Viscometer VT 550 with cooling/heating vessel and sensor systems according to DIN 53019 at a shear rate of 12.9 s⁻¹.

The examples which follow are intended to illustrate the subject matter of the invention without restricting it thereto.

### Examples

### Materials

Capryloyl/Caproyl Anhydro Methyl Glucamide is a mixture of compounds of Formula (I) wherein R is -(CH₂)₈CH₃ and compounds of Formula (I) wherein R is - (CH₂)₆CH₃.

Lauroyl/Myristoyl Methyl Glucamide is a mixture of compounds of Formula (II) wherein R1 is -(CH₂)₁₂CH₃ and compounds of Formula (II) wherein R1 is - (CH₂)₁₀CH₃.

### Example 1:

Assessment of test materials on mucosa *in vitro* tissue models

### Definition

PBS refers to phosphate buffer saline.

### Experimental design

### Cultivation:

Mucosal tissue models were cultivated in 900 µl of culture medium in a 6-well plate.

### Irritation test:

Tissue models were treated with test material. 3 models were used per substance. 90 µl of each test material was applied on the surface of the tissue models. The irritation test was performed in two runs: run 1, substances according to Examples 1c, 1d, 1e and 1f and in run 2, substances according to Examples 1g and 1h, with a 2 minutes interval between each substance.

After substance incubation of 10 minutes at room temperature models were washed. Each model was washed in 3 cups of 50 ml PBS. The model was immersed in the first cup, rotated three times in the cup and the liquid was poured off. The same procedure was followed for cups 2 and 3 and transferred to a fresh prepared 6-well plate with culture medium. For test material according to Example 1h and the positive control, an interval of 3 minutes was set and the samples were rinsed twice with 500 µl PBS before washing with the three cups to remove the viscous substances. In order to avoid remaining substances on the tissue surface, the additional washing step was carried out for these two viscous test materials.

### MTT assay:

Tissue viability was assessed 24 hours after exposure using the MTT assay. Models were transferred to a 24-well plate with 200 µl of MTT solution (1 mg/ml) per well pre-warmed at 37 °C for 30 minutes. Tissue models were incubated in MTT solution for 3 hours at 37 °C, 5% CO₂ and 95% humidity. For extraction, 500 µl of isopropanol was prepared per well of a 24-well plate, the models were transferred and a further 500 µl was added to the insert. The plate was sealed with parafilm and incubated on a shaker for 30 minutes. 1000 µl was then pipetted into the well. From this, 200 µl was transferred to a 96-well plate and the optical density was measured at 570 nm.

### Methods

### Oral in vitro tissue model:

The test was performed using a human oral tissue model. The oral model is a three-dimensional human oral tissue construct composed of normal human keratinocytes. It replicates the structure and function of the natural stratified squamous epithelium of the oral cavity, with a differentiated architecture including basal, spinous, and granular cell layers.

### Assessment of mucosa tissue viability by MTT reduction:

The assessment of mucosa tissue viability was conducted according to the protocol as in the experimental design. Briefly, liquid test substances were applied (90 µ!) to the surface of the models. After the respective treatment time of 10 minutes models were washed according to the performed irritation test with PBS. Following a possible post-exposure incubation period at 37 °C and 5% CO₂ tissue viability is assessed using the MTT assay. For that mucosal tissue models were placed in 200 µl MTT solution (1 mg/mL) (Sigma Aldrich) for 3 h in a humidified atmosphere with 37 °C and 5% CO₂. The MTT reduction was then quantified by extracting the precipitated blue formazan salt with 2 ml 2-propanol and measuring the optical density of the extract at a wavelength of 570 nm using a spectrophotometer in a 96-well plate. After correcting the data using two wells of 2-propanol (200 µl) as blank the relative tissue viability was calculated for each tissue by normalizing the corrected optical density values to the negative control which was set to 100%.

### Results

The results, in particular the normalized mean optical densities, are given in the following Table:

| Example | Test substance | Normalized mean optical density [%] |
|---|---|---|
| 1a | Negative control (PBS) | 100 |
| 1b | Positive control (Sodium Lauryl Sulfate 5%) | 31 |
| 1c | Capryloyl/Caproyl Anhydro Methyl Glucamide 0.5% | 102 |
| 1d | Capryloyl/Caproyl Anhydro Methyl Glucamide 1% | 99 |
| 1e | Capryloyl/Caproyl Anhydro Methyl Glucamide 1.4% | 97 |
| 1f | Sodium Lauroyl Sarcosinate 1% | 73 |
| 1g | Lauroyl/Myristoyl Methyl Glucamide 1% | 103 |
| 1h | Toothpaste + Capryloyl/Caproyl Anhydro Methyl Glucamide 1:1.5 | 102 |

In conclusion, the compounds used in the present invention show a good mucosa tissue viability.

### Example care formulations

### Sensitive toothpaste

| A | Water | Diluent | Ad 100 % |
|---|---|---|---|
| | Tetrasodium Pyrophosphate | Active | 0.05 % |
| | Laponite XLS | Active | 3.00 % |
| | *Sodium Magnesium Silicate* | | |
| | Sorbitol | Humectant | 20.00 % |
| B | Xanthan Gum | Rheology Modifier | 0.30 % |
| | Polyglykol 400 | Humectant | 2.00 % |
| | *PEG-8* | | |
| | Glycerin | Humectant | 22.82 % |
| C | Xylitol | Flavor | 9.70 % |
| | Sodium Saccharin | Flavor | 0.30 % |
| | Sodium Fluoride | Active | 0.23 % |
| | Phenoxetol | Preservative | 0.60 % |
| | *Phenoxyethanol* | | |
| D | Aristoflex AVS | Rheology Modifier | 0.40 % |
| | *Sodium Acryloyldimethyltaurate*/*VP* | | |
| | *Crosspolymer* | | |
| E | Hydrated Silica | Bulking Agent | 7.00 % |
| | Hydrated Silica | Bulking Agent | 9.00 % |
| F | Velsan Flex | Multifunctional | 2.00 % |
| | *Capryloyl*/*Caproyl Anhydro Methyl Glucamide (and) Water* | | |
| | Mint Flavor | Flavor | 1.00 % |
| | Titanium Dioxide | Color | 0.10 % |
| | Glucotain Plus | Surfactant | 0.50 % |
| | *Capryolyl*/*Caproyl Methyl Glucamide (and) Lauroyl*/*Myristoyl Methyl Glucamide* | | |
| G | Lactic Acid 5% / NaOH 5% | Neutralizer | q.s. pH 6.5 |

### Sensitive mouth wash

| A | Water | Diluent | ad 100 % |
|---|---|---|---|
| B | Xanthan Gum | Rheology Modifier | 0.05 % |
| | Polyglykol 400 | Humectant | 5.00 % |
| | *PEG-8* | | |
| | Glycerin | Humectant | 10.00 % |
| C | Xylitol | Flavor | 10.00 % |
| | Aspartame | Flavor | 0.15 % |
| | Benzyl Alcohol | Preservative | 0.50 % |
| D | Mint Flavor | Flavor | 0.70 % |
| | Velsan Flex | Multifunctional | 1.50 % |
| | *Capryloyl*/*Caproyl Anhydro Methyl Glucamide (and) Water* | | |
| E | Brilliant Blue (1% solution) | Dye | 0.03 % |
| F | Lactic Acid 5% / NaOH 5% | Neutralizer | q.s. pH 6.0 |

### Sensitive mouth spray

| A | Water | Diluent | Ad 100 % |
|---|---|---|---|
| | Glycerin 100% | Humectant | 1.00 % |
| B | Salvia Lavandulifolia Leaf Oil | Flavor | 0.01 % |
| | Mentha piperita (peppermint) Oil | Flavor | 0.02 % |
| | Tocopherol | Anti-oxidant | 0.05 % |
| | Velsan Flex | Multifunctional | 2.00 % |
| | *Capryloyl*/*Caproyl Anhydro Methyl Glucamide (and) Water* | | |
| | Benzoic Acid | Preservative | 0.30 % |
| C | Sodium Saccharin | Flavor | 0.20 % |
| | Bisabolol | Active | 0.30 % |
| E | Lactic Acid 5% / NaOH 5% | Neutralizing Agent | q.s. pH 6.0 |

### Intimate cleansing gel

| A | Water | Diluent | Ad 100 % |
|---|---|---|---|
| | Xanthan Gum | Rheology Modifier | 0.80 % |
| B | EDTA-2 Na | Chelating Agent | 0.10 % |
| | Glycerin 100% | Humectant | 1.00 % |
| C | Melaleuca Alternifolia Leaf Oil | Active | 0.01 % |
| | Velsan Flex | Multifunctional | 2.00 % |
| | *Capryloyl*/*Caproyl Anhydro Methyl Glucamide (and) Water* | | |
| D | Sodium Benzoate | Preservative | 0.30 % |
| E | Lactic Acid 5% / NaOH 5% | Neutralizing Agent | q.s. pH 4.0 |

### Intimate refreshment spray

| A | Water | Diluent | Ad 100 % |
|---|---|---|---|
| | Pentylene Glycol | Humectant | 1.00 % |
| | Rosa Damascena Flower Water | Humectant | 2.00 % |
| B | Rosa Damascena Flower Oil | Fragrance | 0.02 % |
| | Velsan Flex | Multifunctional | 1.00 % |
| | *Capryloyl*/*Caproyl Anhydro Methyl Glucamide (and) Water* | | |
| C | Sodium Benzoate | Preservative | 0.30 % |
| D | Lactic Acid 10% / NaOH 5% | Neutralizing Agent | q.s. pH 4.0 |

### Sensitive lubricant

| | | | |
|---|---|---|---|
| A | Water | Diluent | Ad 100 % |
| | Propanediol | Humectant | 1.00 % |
| | Aloe Barbadensis Leaf Juice | Humectant | 1.00 % |
| | Potassium Lactate | Stabilizer | 0.10 % |
| B | Plantasens Biogum Tara | Rheology Modifier | 1.50 % |
| | *Caesalpinia Spinosa Gum* | | |
| C | Velsan Flex | Multifunctional | 1.00 % |
| | *Capryloyl*/*Caproyl Anhydro Methyl Glucamide (and) Water* | | |
| | Benzoic Acid | Preservative | 0.30 % |
| D | Lactic Acid 10% / NaOH 5% | Neutralizing Agent | q.s. pH 4.5 |

### Cleanser for hard contact lenses

| | | | |
|---|---|---|---|
| A | Water | Diluent | Ad 100 % |
| | NaCl | Electrolyte | 0.70 % |
| | Na-Phosphate | Stabilizer | 0.10 % |
| B | Velsan Flex | Multifunctional | 2.00 % |
| | *Capryloyl*/*Caproyl Anhydro Methyl Glucamide (and) Water* | | |
| C | Nipaguard DMDMH Plus | Preservative | 0.20 % |
| | *DMDM Hydantoin* | | |
| D | Citric Acid/ NaOH 10% | Neutralizing Agent | q.s. pH 5.5 |

### Breathe easy congestion relief

| | | | |
|---|---|---|---|
| A | Maris Aqua (Sea) Water isotonic solution | Diluent | Ad 100 % |
| | Aloe Barbadensis Leaf Juice | Humectant | 1.00 % |
| B | Mountain pine essential oil | Active | 0.01 % |
| | *Pinus Mugo Twig Oil* | | |
| C | Velsan Flex | Multifunctional | 0.50 % |
| | *Capryloyl*/*Caproyl Anhydro Methyl Glucamide (and) Water* | | |
| D | Nipagin M Sodium | Preservative | 0.30 % |
| | *Sodium Methyl Paraben* | | |
| E | Citric Acid 10% / NaOH 5% | Neutralizing Agent | q.s. pH 6.0 |

### Lubricant for medical devices

| | | | |
|---|---|---|---|
| A | Water | Diluent | Ad 100 % |
| | Propanediol | Humectant | 1.00 % |
| B | Motusflex MF *Sodium Polyacryloyldimethyl Taurate* | Rheology Modifier | 1.50 % |
| C | Velsan Flex | Multifunctional | 1.00 % |
| | *Capryloyl*/*Caproyl Anhydro Methyl Glucamide (and) Water* | | |
| | Benzoic Acid | Preservative | 0.30 % |
| D | Citric Acid 10% / NaOH 5% | Neutralizing Agent | q.s. pH 5.0 |

### Micellar cleansing foam for pets

| | | | |
|---|---|---|---|
| A | Water | Diluent | Ad 100 % |
| | E DTA-2 N a | Chelating Agent | 0.10 % |
| | Glycerin 100% | Humectant | 1.00 % |
| B | Polyquaternium-10 | Conditioning Agent | 0.10 % |
| C | Velsan Flex | Multifunctional | 2.00 % |
| | *Capryloyl*/*Caproyl Anhydro Methyl Glucamide (and) Water* | | |
| D | Sodium Benzoate | Preservative | 0.50 % |
| E | Citric Acid / NaOH 10% | Neutralizing Agent | q.s. pH 5.0 |

### Dental spray (clean and care) for dogs

| | | | |
|---|---|---|---|
| A | Water | Diluent | Ad 100 % |
| | Chamomilla Recutita Flower Extract | Active | 0.20 % |
| | *Glycerin, Aqua, Chamomilla Recutita (Matricaria) Flower Extract* | | |
| B | Salvia Triloba (Sage) Leaf Extract (and) Helianthus Annuus (Sunflower) Seed Oil | Active | 0.05 % |
| | Velsan Flex | Multifunctional | 1.00 % |
| | *Capryloyl*/*Caproyl Anhydro Methyl Glucamide (and) Water* | | |
| C | Phenoxetol | Preservative | 0.80 % |
| | *Phenoxyethanol* | | |
| D | Citric Acid / NaOH 10% | Neutralizing Agent | q.s. pH 6.0 |

### Anti plaque toothpaste for cats and dogs

| | | | |
|---|---|---|---|
| A | Water | Diluent | Ad 100 % |
| | Aristoflex Silk | Rheology Modifier | 1.00 % |
| | *Sodium Polyacryloyldimethyl Taurate* | | |
| | Sorbitol 70% | Humectant | 50.00 % |
| B | Salvia Triloba (Sage) Leaf Extract (and) Helianthus Annuus (Sunflower) Seed Oil | Active | 0.05 % |
| | Velsan Flex | Multifunctional | 2.00 % |
| | *Capryloyl*/*Caproyl Anhydro Methyl Glucamide (and) Water* | | |
| | Flavoring Oil | Flavor | 0.20 % |
| C | Nipaguard SCE | Preservative | 1.30 % |
| | *Sorbitan Caprylate (and) Propanediol (and) Benzoic Acid* | | |
| D | Hydrated Silica | Bulking Agent | 22.00 % |
| E | Citric Acid / NaOH 10% | Neutralizing Agent | q.s. pH 6.0 |

### Ear and eye cleanser for pets

| | | | |
|---|---|---|---|
| A | Water | Diluent | Ad 100 % |
| | NaCl | Electrolyte | 0.70 % |
| | Na-Phosphate | Stabilizer | 0.10 % |
| B | Velsan Flex | Multifunctional | 0.50 % |
| | *Capryloyl*/*Caproyl Anhydro Methyl Glucamide (and) Water* | | |
| C | Nipaguard DMDMH Plus | Preservative | 0.20 % |
| | *DMDM Hydantoin* | | |
| D | Citric Acid / NaOH 10% | Neutralizing Agent | q.s. pH 6.5 |

## Claims

1. Use of compound X selected from
compounds of Formula (I) 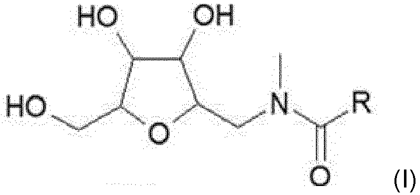
wherein R is selected from saturated hydrocarbon chains having 5 to 23 carbon atoms, unsaturated hydrocarbon chains having 5 to 23 carbon atoms, and mixtures thereof,
compounds of Formula (II) 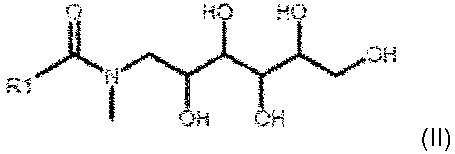
wherein R1 is selected from saturated hydrocarbon chains having 5 to 23 carbon atoms, unsaturated hydrocarbon chains having 5 to 23 carbon atoms, and mixtures thereof,
glyceryl ethers, sorbitan esters, and isosorbide esters
on mucosal tissue.

2. The use according to claim 1, wherein the mucosal tissue is selected from oral mucosal tissue, intimate mucosal tissue, eye mucosal tissue, ear mucosal tissue (including eardrum), and nasal mucosal tissue.

3. The use according to claim 1 or 2, wherein compound X is selected from compounds of Formula (I) and compounds of Formula (II).

4. The use according to any of claims 1 to 3, wherein
R in Formula (I) is selected from saturated hydrocarbon chains having 5 to 17 carbon atoms, unsaturated hydrocarbon chains having 5 to 17 carbon atoms, and mixtures thereof,
preferably is selected from saturated hydrocarbon chains having 5 to 13 carbon atoms, unsaturated hydrocarbon chains having 5 to 13 carbon atoms, and mixtures thereof,
particularly preferably is selected from saturated hydrocarbon chains having 7 to 9 carbon atoms, unsaturated hydrocarbon chains having 7 to 9 carbon atoms, and mixtures thereof.

5. The use according to any of claims 1 to 4, wherein
R1 in Formula (II) is selected from saturated hydrocarbon chains having 5 to 17 carbon atoms, unsaturated hydrocarbon chains having 5 to 17 carbon atoms, and mixtures thereof,
preferably is selected from saturated hydrocarbon chains having 7 to 13 carbon atoms, unsaturated hydrocarbon chains having 7 to 13 carbon atoms, and mixtures thereof,
particularly preferably is selected from saturated hydrocarbon chains having 11 to 13 carbon atoms, unsaturated hydrocarbon chains having 11 to 13 carbon atoms, and mixtures thereof.

6. The use according to any of claims 1 to 5, wherein the glyceryl ethers are mono- or diethers of glycerin and one or more C6-C20 fatty alcohols, preferably mono- or diethers of glycerin and one or more C8-C14 fatty alcohols, particularly preferably monoethers of glycerin and one or more C8 fatty alcohols.

7. The use according to any of claims 1 to 6, wherein the sorbitan esters are mono-, di- or triesters of sorbitan and one or more C6-C20 fatty acids, preferably mono- or diesters of sorbitan and one or more C8-C14 fatty acids, particularly preferably mono- or diesters of sorbitan and caprylic acid.

8. The use according to any of claims 1 to 7, wherein the isosorbide esters are mono- or diesters of isosorbide and one or more C6-C20 fatty acids, preferably mono- or diesters of isosorbide and one or more C8-C14 fatty acids, particularly preferably mono- or diesters of isosorbide and caprylic acid.

9. Compound X as defined in any of claims 1 to 8 for use on mucosal tissue.

10. A care formulation comprising
from 0.1 to 20 wt.-%, preferably from 0.2 to 10 wt.-%, more preferably from 0.3 to 5 wt.-%, even more preferably from 0.4 to 3 wt.-%, particularly preferably from 0.5 to 2 wt.-%, for example from 0.8 to 1.5 wt.-%, based on the total weight of the care formulation, of compound X as defined in any of claims 1 to 8,
wherein the care formulation is selected from an oral care formulation, intimate care formulation, eye care formulation, ear care formulation, nasal care formulation, and lubricant for medical devices or medical tools.

11. The care formulation according to claim 10, wherein the care formulation is for human care or animal care.

12. The care formulation according to claim 10 or 11, wherein the oral care formulation is selected from a toothpaste, oral gel, mouth wash, tooth powder, oral spray, oral care wipes, dental wipes, dental floss, and chewing gum.

13. The care formulation according to any of claims 10 to 12, wherein the intimate care formulation is selected from an intimate cleansing formulation, intimate deodorant, intimate lubricant, and intimate wipes.

14. Use of a care formulation as defined in any of claims 1 to 8 on mucosal tissue.

15. Care formulation as defined in any of claims 1 to 8 for use on mucosal tissue.
